**Europäisches Patentamt**

(19) **European Patent Office**

**Office européen des brevets**

(11) Veröffentlichungsnummer: **0 024 489**
**B1**

(12) # EUROPÄISCHE PATENTSCHRIFT

(45) Veröffentlichungstag der Patentschrift:
14.04.82

(51) Int. Cl.³: **C 07 C  87/30**, C 07 C  85/04

(21) Anmeldenummer: **80103434.9**

(22) Anmeldetag: **20.06.80**

(54) **Verfahren zur Gewinnung farbloser, Trimethylamin- und Dichlorethan-freier wässriger Chlorcholinchloridlösungen.**

(30) Priorität: **25.08.79  DE 2934495**

(43) Veröffentlichungstag der Anmeldung:
**11.03.81 Patentblatt 81/10**

(45) Bekanntmachung des Hinweises auf die Patenterteilung:
**14.04.82 Patentblatt 82/15**

(84) Benannte Vertragsstaaten:
**AT FR GB IT NL**

(56) Entgegenhaltungen:
**DD-A-101 142**

(73) Patentinhaber: **CHEMISCHE WERKE HÜLS AG,
Postfach 1320, D-4370 Marl 1 (DE)**

(72) Erfinder: **Grasemann, Horst, Dr., Von-Menzel-Strasse 12,
D-4370 Marl (DE)**
Erfinder: **Müller, Wolfgang Hans Eduard, Dr., Bitterfelder
Strasse 9a, D-4370 Marl (DE)**

## Verfahren zur Gewinnung farbloser, Trimethylamin- und Dichlorethan-freier wässriger Chlorcholinchloridlösungen

Chlorcholinchlorid kann man durch Umsetzung von Trimethylamin mit Dichlorethan in einer Lösung von Toluol herstellen [J. biol. chemistry 235, Seite 475 bis 479 (1960)]. Diese Umsetzung erfolgt jedoch auch bei hohen Temperaturen sehr langsam. Führt man die Reaktion bei 120°C in dieser Lösung durch, dann sind nach 7 Stunden erst etwa 80% der Einsatzstoffe umgesetzt. Durch die lange Reaktionszeit, in Verbindung mit der hohen Temperatur, steigt zudem die Nebenausbeute an Trimethylammoniumchlorid stark an. Diese unerwünschte Verbindung entsteht auch, wenn man mit wässrigem Trimethylamin arbeitet (DE-PS 1 543 341 = US-PS 3 557 214).

Durch einen Übeschuss an Dichlorethan kann man die Reaktionsgeschwindigkeit erhöhen. Verwendet man Dichlorethan mit mindestens 4- bis 6fachem Überschuss, bezogen auf wasserfreies Trimethylamin, dann läuft die Reaktion bei etwa 100°C unter Eigendruck innerhalb von 1,5 bis 2 Stunden bis zu einem 98prozentigem Umsatz ab (DE-PS 1 275 066).

Nach der Lehre dieser Patentschrift kann man das in überschüssigem Dichlorethan suspendierte Chlorcholinchlorid auf zweierlei Weise gewinnen:

Nach dem ersten Weg gibt man den auf 20 bis 25°C abgekühlten Reaktorinhalt auf eine Zentrifuge und trennt auf diese Weise den Feststoff Chlorcholinchlorid vom flüssigen Dichlorethan. Die Trennung ist jedoch nicht quantitativ, so dass das schleuderfeuchte Chlorcholinchlorid in einer Trocknungsanlage nachgetrocknet werden muss. Durch eine aufwendige Brüdenkondensation müssen die geringen Mengen Dichlorethan aufgefangen werden.

Eleganter ist die zweite Art der Aufarbeitung durch Zugabe von Wasser zum vorgekühlten Reaktorinhalt, zumal das Chlorcholinchlorid ohnehin als wässrige Lösung gehandelt wird. Beide Flüssigkeiten trennt man über eine Trennflasche. Die abgetrennte wässrige Chlorcholinchloridlösung enthält noch Trimethylamin und Dichlorethan.

Um sie vom Trimethylamin und Dichlorethan zu befreien, destilliert man die wässrige Chlorcholinchloridlösung im Vakuum an. Hierbei färbt sich die bis dahin farblose wässrige Chlorcholinchloridlösung jedoch gelb und der pH-Wert sinkt von 6 auf 1, da Salzsäure frei wird. Diese Salzsäure bewirkt erhebliche Korrosionsprobleme. Ausserdem steigt die Konzentration von unerwünschtem Trimethylammoniumchlorid von 9,8 g/l 70prozentige Lösung auf 20 g/l in der 70prozentigen Lösung.

Damit stellt sich die Aufgabe, ein Verfahren zu finden, mit dem man in einfacher Weise farblose, Trimethylamin- und Dichlorethan-freie wässrige Chlorcholinchloridlösungen erhält.

Diese Aufgabe wird erfindungsgemäss dadurch gelöst, dass man die wässrige Trimethylamin- und Dichlorethan-haltige Chlorcholinchloridlösung unter vermindertem Druck bei Temperaturen von 20 bis 70°C innerhalb kurzer Verweilzeit kontinuierlich im Gegenstrom in einer Kolonne mit 0,2 bis 20 Gewichtsprozent Wasserdampf, bezogen auf die zu reinigende Chlorcholinchloridlösung, behandelt. Diese kontinuierliche Behandlung im Gegenstrom mit Wasserdampf führt man vorzugsweise in einer Kolonne mit ein bis zehn praktischen Trennstufen durch. Die Behandlung mit geringen Mengen Wasserdampf erfolgt vorteilhaft in einer Kolonne mit mehr als 10 praktischen Trennstufen. Bevorzugt arbeitet man bei Temperaturen von 40 bis 60°C. Die Verweilzeit beträgt < 1 Stunde, bevorzugt 1 bis 10 Minuten. Wesentlich für die erfindungsgemässe Reinigung ist die Durchführung bei vermindertem Druck, vorzugsweise bei 0,02 bis 0,9 bar, insbesondere bei 0,04 bis 0,2 bar. Besonders gute Ergebnisse unter äusserst wirtschaftlichen Bedingungen erzielt man beim Einsatz von 0,5 bis 10 Gewichtsprozent Wasserdampf, bezogen auf die zu reinigende wässrige Chlorcholinchloridlösung.

Überraschenderweise ist weder eine Verfärbung noch ein Absinken des pH-Wertes zu beobachten, wenn man Dichlorethan- und Trimethylamin-haltige wässrige Chlorcholinchloridlösung bei vermindertem Druck, beispielsweise bei 0,05 bis 0,1 bar, durch eine Kolonne, beispielsweise eine Kolonne mit fünf praktischen Böden, rieseln lässt, wobei man die Flüssigkeit mit Wasserdampf behandelt. Die abgezogene Chlorcholinchloridlösung ist farblos und frei von Trimethylamin und Dichlorethan. Sie hat einen pH-Wert von 5,8.

Beispiel 1

Einen druckfesten 1 m³-Reaktor, versehen mit Rührer, Thermometer, Manometer und einem Überdruckventil, füllt man mit 830 kg Dichlorethan. Durch eine Mantelheizung heizt man das System auf 60°C auf. Danach pumpt man 55 kg verflüssigtes Trimethylamin in den geschlossenen Reaktor. Innerhalb von 30 Minuten dosiert man diese Menge Trimethylamin so zu, dass die Reaktion bei 120°C und einem Druck von 2,4 bar abläuft. Nach einer Stunde ist ein Umsatz von 95%, bezogen auf das eingesetzte Trimethylamin, erzielt. Man kühlt auf 80°C ab und gibt unter Rühren 65 kg Wasser dazu. Die im überschüssigen Dichlorethan suspendierten Kristalle lösen sich spontan, und es kommt zur Ausbildung von zwei Phasen. Die untere besteht im wesentlichen aus nicht umgesetztem Dichlorethan, die obere aus 210 kg einer wässrigen Chlorcholinchloridlösung mit einem Gehalt von 70 Gewichtsprozent dieses Wirkstoffes. In der wässrigen 70prozentigen Chlorlolinchloridlösung sind ausserdem enthalten:

9,8 g/l Trimethylammoniumhydrochlorid
2,38 g/l Trimethylamin
6 g/l Dichlorethan

Der pH-Wert ist 10. Die APHA-Farbzahl beträgt 5. Das abgetrennte Dichlorethan enthält 4 g/l Trimethylamin.

Die 210 kg 70prozentige, mit Trimethylamin und Dichlorethan verunreinigte Chlorcholinchloridlösung wird bei einem Unterdruck von 0,1 bar mit einer Geschwindigkeit von 2 kg/h durch eine Kolonne mit 5 praktischen Böden und 80 mm Durchmesser gegeben. Die Aufgabe erfolgt am Kopf der Kolonne. Im Gegenstrom bläst man vom unteren Ende des Rohres 0,17 kg Wasserdampf/h durch. Die Temperatur in der Kolonne beträgt 57 bis 59°C.

Die am Bodfen ablaufende Chlorcholinchloridlösung hat sich in der Farbzahl nicht verändert und ist frei von Trimethylamin und Dichlorethan. Der pH-Wert beträgt 5,8.

Vergleichsbeispiel 2

Zum Vergleich rektifiziert man die 210 kg der nach den Angaben des Beispiels 1 hergestellten Chlorcholinchloridlösung in konventioneller Weise bei einem Druck von ebenfalls 0,1 bar. Nach Abtrennung des Dichlorethans und des Trimethylamins erhält man eine Chlorcholinchloridlösung mit einer APHA-Farbzahl von 80 und einem pH-Wert von 1.

Beispiel 3

Man arbeitet nach den Angaben des Beispiels 1, wendet jedoch zur Reinigung der 70prozentigen Chlorcholinchloridlösung nur 0,1 kg Wasserdampf/h bei einer Belastung von 2 kg/h Chlorcholinchloridlösung an und erhält eine farblose Chlorcholinchloridlösung mit unveränderter Farbzahl von APHA 5, mit 7 Gew. ppm Dichlorethan und kaum nachweisbarem Trimethylamingehalt.

Beispiel 4

Man verfährt wie in Beispiel 1 beschrieben, wendet jedoch zur Reinigung der 70prozentigen Chlorcholinchloridlösung eine Kolonne mit 10 praktischen Böden an. Bei einer Wasserdampfmenge von 0,1 kg Wasserdampf/h und einer Belastung von 2 kg Chlorcholinchloridlösung/h ist weder Dichlorethan noch Trimethylamin in der erhaltenen Chlorcholinchloridlösung nachweisbar. Die Lösung ist farblos; die Farbzahl ist unverändert.

Beispiel 5

Man arbeitet nach den Angaben des Beispiels 1, führt die Wasserdampfbehandlung jedoch bei einer Temperatur der Lösung von 24°C und einem Druck von 0,027 bis 0,029 bar durch. Man erhält wie im Beispiel 1 eine farblose Lösung, in der weder Dichlorethan noch Trimethylamin nachweisbar sind.

Beispiel 6

Man arbeitet nach den Angaben des Beispiels 1, führt die Wasserdampfbehandlung jedoch bei einer Temperatur in der Kolonne von 70°C und einem Druck von 0,3 bar durch. Man erhält eine farblose Lösung, die frei von Trimethylamin und Dichlorethan ist.

Beispiel 7

Man arbeitet nach den Angaben des Beispiels 1, führt die Wasserdampfbehandlung jedoch in einer Kolonne mit 25 praktischen Böden mit 0,5% Wasserdampf, bezogen auf die zu reinigende Lösung, durch. Man erhält eine farblose Lösung, die nur 2 ppm Dichlorethan und kein Trimethylamin enthält.

**Pantentansprüche**

1. Verfahren zur Gewinnung farbloser, Trimethylamin- und Dichlorethan-freier wässriger Chlorcholinchloridlösungen durch Umsetzung von Trimethylamin mit überschüssigem Dichlorethan und Auflösen der entstandenen Chlorcholinchloridkristalle in Wasser, Trennung der entstehenden Mischung in eine dichlorethanreiche und eine wässrige chlorcholinchloridreiche Phase, die noch Dichlorethan und Trimethylamin enthält, dadurch gekennzeichnet, dass man die wässrige Triemthylamin- und Dichlorethan-haltige Chlorcholinchloridlösung unter vermindertem Druck bei Temperaturen von 20 bis 70°C innerhalb kurzer Verweilzeit kontinuierlich im Gegenstrom in einer Kolonne mit 0,2 bis 20 Gewichtsprozent Wasserdampf, bezogen auf die zu reinigende Chlorcholinchloridlösung, behandelt.

2. Verfahren nach Anspruch 1, dadurch gekennzeichnet, dass man die Wasserdampfbehandlung kontinuierlich im Gegenstrom in einer Kolonne mit 1 bis 10 praktischen Trennstufen vornimmt.

3. Verfahren nach den Ansprüchen 1 und 2, dadurch gekennzeichnet, dass man bei Temperaturen von 40 bis 60°C arbeitet.

4. Verfahren nach den Ansprüchen 1 bis 3, dadurch gekennzeichnet, dass man eine Wasserdampfmenge von 0,5 bis 10 Gewichtsprozent Wasserdampf, bezogen auf die zu reinigende wässrige Chlorcholinchloridlösung, anwendet.

5. Verfahren nach den Ansprüchen 1 bis 4, dadurch gekennzeichnet, dass man bei einem Druck von 0,02 bis 0,9 bar, vorzugsweise bei 0,04 bis 0,2 bar Druck arbeitet.

**Claims**

1. A process for obtaining a colourless aqueous chlorocholine chloride solution free from trimethylamine and dichloroethane by reaction of trimethylamine with excess dichloroethane, dissolution of the resulting chlorocholine chloride crystals in water, and separation of the resulting mixture into a dichloroethane-rich phase and an aqueous chlorocholine chloride-rich phase which still contains dichloroethane and trimethylamine, characterised in that the aqueous chlorocholine chloride solution containing dichloroethane and trimethylamine is treated at reduced pressure and a temperature of 20 to 70°C with from 0.2 to 20 percent by weight of steam, based on the chlorocholine chloride solution to be purified, continuously in countercurrent in a column with a short residence time.

2. A process according to claim 1, characterised in that the treatment with steam is carried out continuously in countercurrent in a column with 1 to 10 practical separating stages.

3. A process according to claims 1 and 2, characterised in that a temperature of 40 to 60°C is used.

4. A process according to claims 1 to 3, characterised in that an amount of 0.5 to 10 percent by weight of steam, based on the aqueous chlorocholine chloride to be purified, is used.

5. A process according to claims 1 to 4, characterised in that a pressure of 0.02 to 0.9 bar, preferably 0.04 to 0.2 bar, is used.

**Revendications**

1. Procédé pour l'obtention de solutions aqueuses incolores de chlorure de chlorocholine qui sont exemptes de triméthylamine et de dichloréthane, par réaction de triméthylamine sur du dichloréthane en excès et dissolution dans de l'eau des cristaux de chlorure de chlorocholine qui se sont formés, par séparation du mélange qui se forme en une phase riche en dichloréthane et en une phase aqueuse riche en chlorure de chlorocholine renfermant encore du dichloréthane et de la triméthylamine, ledit procédé étant caractérisé par le fait qu'on traite la solution aqueuse de chlorure de chlorocholine, renfermant de la triméthylamine et du dichloréthane, sous pression réduite, à des températures de 20 à 70°C, en continu, à contre-courant dans une colonne, par 0,2 à 20% en poids de vapeur d'eau, rapporté à la solution de chlorure de chlorocholine à purifier.

2. Procédé selon la revendication 1, caractérisé par le fait qu'on effectue le traitement par la vapeur d'eau en continu et à contre-courant dans une colonne comportant de un à 10 étages effectifs de séparation.

3. Procédé selon les revendications 1 et 2, caractérisé par le fait qu'on travaille à des températures de 40 à 60°C.

4. Procédé selon les revendications 1 à 3, caractérisé par le fait qu'on utilise une quantité de vapeur d'eau de 0,5 à 10% en poids, rapporté à la solution aqueuse de chlorure de chlorocholine à purifier.

5. Procédé selon les revendications 1 à 4, caractérisé par le fait qu'on travaille à une pression de 0,02 à 0,9 bar, de préférence de 0,04 à 0,2 bar.